# EUROPEAN PATENT SPECIFICATION

(11) **EP 4 154 875 B1**
(45) Date of publication and mention of the grant of the patent: **20.11.2024**
(21) Application number: 22194900.1
(22) Date of filing: 09.09.2022
(51) Int. Cl.: A61K 31/164, A61K 9/00, A61K 31/202, A61P 25/24, A61P 25/00, A61K 9/08, A61K 9/16, A61K 47/26, A61K 9/48

(54) **N-PALMITOYLETHANOLAMIDE AND DOCOSAHEXAENOIC ACID FOR USE IN THE TREATMENT OF AUTISM SPECTRUM DISORDER AND OTHER DEPRESSIVE SYNDROMES**
N-PALMITOYLETHANOLAMID UND DOCOSAHEXAENSÄURE ZUR VERWENDUNG BEI DER BEHANDLUNG VON AUTISMUS-SPEKTRUM-STÖRUNG UND ANDEREN DEPRESSIVEN SYNDROMEN
N-PALMITOYLÉTHANOLAMIDE ET ACIDE DOCOSAHEXAÉNOÏQUE À UTILISER DANS LE TRAITEMENT DE TROUBLES DU SPECTRE AUTISTIQUE ET D'AUTRES SYNDROMES DÉPRESSIFS

(30) Priority: 23.09.2021 IT 202100024464
(43) Date of publication of application: 29.03.2023
(73) Proprietor: EPITECH GROUP S.p.A., 20144 Milano (MI) (IT)
(72) Inventor: DELLA VALLE, Francesco, deceased (IT); DELLA VALLE, Maria Federica, I-20144 MILAN (IT); GOMIERO, Chiara, I-20144 MILAN (IT); MARCOLONGO, Gabriele, I-20144 MILAN (IT); CALIGNANO, Antonio, I-20144 MILAN (IT); CRISTIANO, Claudia, I-20144 MILAN (IT)
(74) Representative: Long, Giorgio

(56) References cited:
- WO-A1-2016/020828
- US-A1- 2011 237 669
- PERITORE ALESSIO FILIPPO ET AL: "Therapeutic Efficacy of Palmitoylethanolamide and Its New Formulations in Synergy with Different Antioxidant Molecules Present in Diets", NUTRIENTS, vol. 11, no. 9, 11 September 2019 (2019-09-11), pages 2175, XP055914112, DOI: 10.3390/nu11092175
- RUSSO ROBERTO ET AL: "Gut-brain Axis: Role of Lipids in the Regulation of Inflammation, Pain and CNS Diseases", CURRENT MEDICINAL CHEMISTRY, vol. 25, no. 32, 16 October 2018 (2018-10-16), NL, pages 3930 - 3952, XP055794155, ISSN: 0929-8673, DOI: 10.2174/0929867324666170216113756
- ZOU MINGYANG ET AL: "Role of the endocannabinoid system in neurological disorders", INTERNATIONAL JOURNAL OF DEVELOPMENTAL NEUROSCIENCE, PERGAMON, OXFORD, GB, vol. 76, 8 March 2019 (2019-03-08), pages 95 - 102, XP085800921, ISSN: 0736-5748, [retrieved on 20190308], DOI: 10.1016/J.IJDEVNEU.2019.03.002

## Description

### Technical field of the invention

The present invention relates to the use of N-palmitoylethanolamide (PEA) in combination with docosahexaenoic acid (DHA) in the treatment of autism spectrum disorder and other depressive syndromes.

### Background art

Autism spectrum disorder (ASD) is a multifactorial-etiology neurodevelopmental disorder which manifests through the impairment of social interaction, verbal and non-verbal communication, activities, and interest (definition from the *American Psychiatric Association).* The symptomatology begins within the first three years of life following a specific event and early diagnosis is very important to be able to adequately intervene on the various compromised areas.

Stereotypes are one of the main diagnostic features of ASD and can comprise self-stimulatory and self-harming behaviors, excitement, stimulation, stress, anxiety, boredom, fatigue, and social isolation.

Although the cause is not known, the appearance of ASD is also due to an imbalance between inhibitory and excitatory synapses, as well as neuroinflammation responsible for the activation and pathological proliferation of non-neuronal cells which, by releasing cytokines (IL-1α, IL-1β, IL-6 and TNF-α) and pro-inflammatory chemokines (MCP-1 and RANTES) in the brain and cerebrospinal fluid of autistic patients, exacerbate the neuroinflammatory process.

It is further known that a class of endogenous hormones, known as neurosteroids, are involved in ASD: the most important is allopregnanolone (ALLO), a potent metabolite of progesterone and modulator of GABA_{A} receptors synthesized by 5α-reductase type 1 enzymes and 3α-hydroxysteroid dehydrogenase. ALLO possesses antidepressant, anxiolytic, anti-stress, sedative, anti-aggressive and analgesic properties and prevents the formation and release of pro-inflammatory cytokines, such as NFkB, HMGB1, MCP-1 and TNF-a involved in multiple neuroinflammatory conditions.

The reduction in plasma and brain ALLO levels found in patients with ASD is strongly correlated with the severity of autistic symptomatology.

The drugs authorized and administered to patients with ASD are *risperidone* and *methylphenidate,* which, although useful for treating autistic symptoms, do not act on the modulation of neuroinflammation and on the increase in endogenous ALLO levels.

A drastic reduction in endogenous ALLO levels has also been found in psychiatric conditions such as major depressive disorder (MDD), post-traumatic stress disorder (PTSD) and especially in postpartum depression.

Only in 2019, ALLO, known as Brexanolone, was placed on the market in the United States under the name of Zulresso^{™} as an intravenous infusion to be administered over 2.5 days. Zulresso^{™} is the first drug approved by the FDA (Federal Drug Administration) for its exclusive use in the treatment of postpartum depression. In 2016, the EMA included Brexanolone in a study program prior to its marketing in Europe.

Although it is declared an effective and safe drug, Zulresso^{™} should only be administered in certified healthcare facilities due to the risk of excessive sedation, sudden loss of consciousness or dizziness during the 60 hours of administration.

To date, there are no clinical studies in which ALLO is administered as a therapy for autistic patients: it is thus of considerable importance to identify an anti-neuroinflammatory treatment which is easy to administer orally, safe and free of significant side effects in the long term and capable of also normalizing endogenous ALLO levels.

A natural mechanism for modulating neuroinflammation is the endogenous molecule Palmitoylethanolamide (PEA). In pre-clinical and clinical settings, the administration of PEA, especially if in an ultra-micronized form (PEA-um), is capable of determining neuroinflammatory normalization activity; in particular, it has been demonstrated that PEA-um is capable of significantly sub-modulating the general neuroinflammatory status of mice with a simil-autistic phenotype, reducing the expression of the pro-inflammatory hippocampal and serum cytokines IL-6, IL-1b and TNF-a and modulating the altered behavioral status. Clinically, the 3-month treatment of autistic children with PEA-um 600 mg/day improves aggressiveness, cognitive and behavioral skills, and communication without adverse effects.

DHA (C22:6 n-3) is one of the most abundant long-chain polyunsaturated fatty acids (PUFAs) in the body. It is a fundamental component of all cell membranes, including the cells of the nervous system. The decrease thereof can trigger a malfunction of nervous tissue, negatively affecting learning and behavioral processes as well as aggravating the autistic disease.

Clinical trials have been conducted in patients with ASD by means of administration of PUFA, typically a mixture of EPA and DHA, at high doses (generally greater than 1 g per day), but the results have been poor and insignificant.

Peritore AF et al (Nutrients, 2019, 11(9): 2175) concerns PEA, optionally in micronized form, for use in the treatment of acute and chronic inflammatory pathologies. Russo R et al (Current Medicinal Chemistry, 2018, 25(32): 3930-52) teaches that the gut-brain axis has been found to play a role in stress-related disorders, to neurodevelopmental disorders and to neurodegenerative diseases. PEA is a type of bioactive lipid that reduces inflammation markers in a murine model of inflammatory bowel disease. DHA is mentioned to be reduced in plasma of children with autism spectrum disorders (ASD) and supplementation with PUFAs improves the symptoms of ASD. US 2011/237669 mentions that DHA is used in the treatment of autism. Zou M et al (International Journal of Developmental Neuroscience, 2019, 76:95-102) discloses that oral supplementation of PEA is used in the treatment of ASD. It also mentions that DHA and other PUFAs are decreased in ASD and that supplementation with n-3 PUFA improves ASD.

Therefore, there is a need to provide ASD therapy which is effective, non-invasive and safe and which, if possible, does not require the administration of high doses of active substances. In fact, taking into account that such therapy would be primarily dedicated to a child population, repeated administration and/or in large dosage forms (for example, large tablets for oral administration of high doses of active ingredients) would be difficult to accept by the patient.

### Summary of the invention

The present invention derives from the surprising discovery that palmitoylethanolamide (PEA), preferably when used in an ultra-micronized form, when administered in combination with docosahexaenoic acid (DHA), exhibits a synergistically relevant effect in improving the behavioral parameters of autistic subjects and in raising endogenous allopregnanolone (ALLO) levels.

Therefore, the present invention relates to palmitoylethanolamide for use in the treatment of autism spectrum disorders (ASDs), where palmitoylethanolamide is administered in association with docosahexaenoic acid, where said administration is separate, combined, or simultaneous.

The invention further relates to a composition containing palmitoylethanolamide and docosahexaenoic acid, in particular when usable in the treatment of autism spectrum disorders (ASD).

The invention also relates to palmitoylethanolamide for use in the treatment of diseases characterized by decreased endogenous allopregnanolone levels, where palmitoylethanolamide is administered in combination with docosahexaenoic acid, where said administration is separate, combined, or simultaneous.

These and further objects, as outlined in the appended claims, will be described in the following description. The text of the claims should be considered included in the description in order to assess the description sufficiency.

Further features and advantages of the invention will become apparent from the following description of preferred embodiments, given by way of non-limiting examples.

### Brief description of the drawings

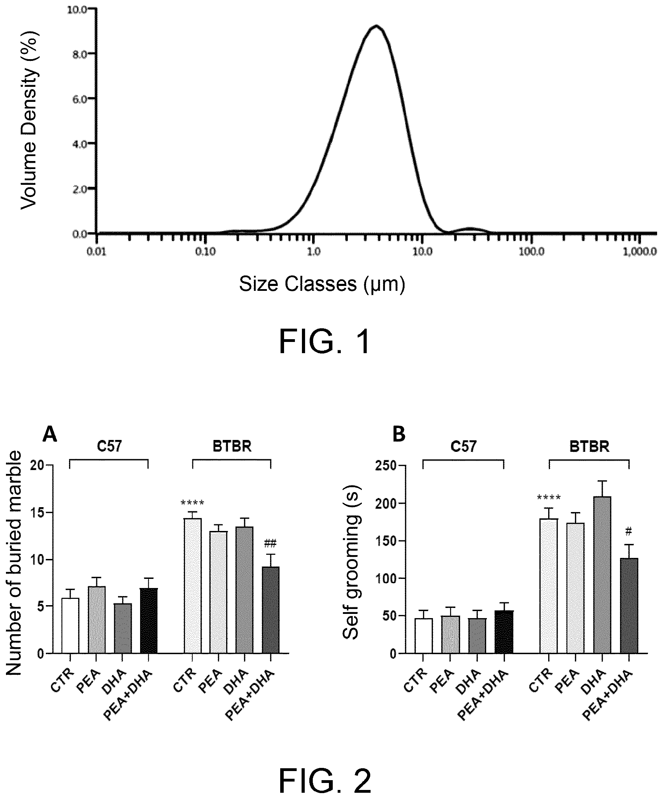

Figure 1 shows a graph of particle size distribution of palmitoylethanolamide in an ultra-micronized form (PEA-um) ;
Figure 2 shows the effect of the synergy of PEA-um associated with DHA on repetitive/obsessive behavior of BTBR mice. (A) Number of marbles buried in 15 min; (B) time spent self-cleaning by C57 and BTBR mice. All values are reported as mean ± SEM of 8 animals for each group. ****p <0.0001 vs. C57 CTR; ^{#}p <0.05 vs. BTBR CTR; ^{##}p <0.01 vs. BTBR CTR;

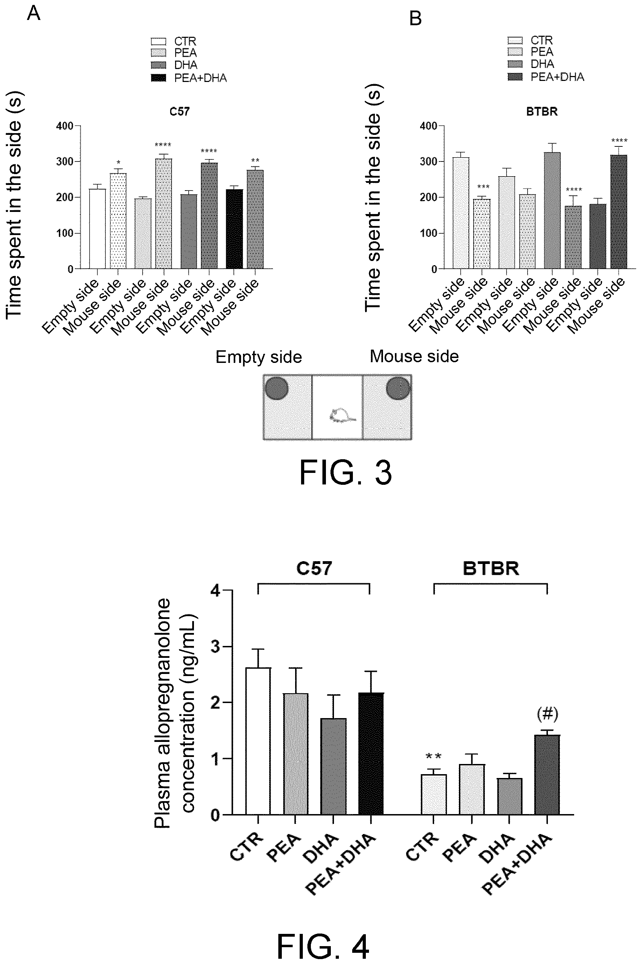

Figure 3 shows the effect of all the treatments on the sociability of mice. (A) Time spent by C57 mice in the empty room or room occupied by a mouse; (B) same assessment for BTBR mice: only treatment with PEA+DHA is capable of improving the sociability of mice. All values are reported as mean + SEM of 8 animals for each group. *p <0.05 vs. C57 CTR; **p <0.01 vs. C57 CTR; ***p <0.001 vs. C57 CTR; ****p <0.0001 vs. C57 CTR;
Figure 4 shows the neurosteroidogenic effect of the synergy between PEA-um and DHA. PEA-um associated with DHA increases plasma ALLO levels of BTBR mice. PEA+DHA does not increase the plasma ALLO levels in C57 mice. All values are reported as mean ± SEM of 8 animals for each group. **p <0.01 vs. C57 CTR; ^{(#)}p <0.05 vs. BTBR CTR.

### Detailed description of the invention

The present invention relates in a first aspect to palmitoylethanolamide (PEA) for use in the treatment of autism spectrum disorders (ASD), where palmitoylethanolamide is administered in association with docosahexaenoic acid (DHA), where said administration is separate, combined, or simultaneous.

The term "in association" means both a combination therapy and a therapy in which PEA and DHA are contained in a single dosage form.

"Separate" administration means an administration of PEA and DHA in separate dosage forms, administered at different times ranging from 1 minute to several hours, for example 8, 12 or 14 hours apart.

"Combined" administration means an administration of PEA and DHA contained in a single dosage form, i.e., a pharmaceutical or veterinary composition or formulation, supplement, dietary composition or food for special medical purposes.

"Simultaneous" administration means an administration of PEA and DHA in separate dosage forms, but administered simultaneously, i.e., within a separation time between the PEA and DHA administration, or vice versa, not exceeding 1 minute.

Palmitoylethanolamide can be administered in any form, for example in a non-micronized form, in a micronized form or in an ultra-micronized form.

The term "palmitoylethanolamide (or PEA) in a non-micronized form" means PEA having a particle size distribution, defined as a percentage by volume and measured with the laser light scattering method, represented by a distribution curve having the mode above 10 microns, preferably above 20 microns.

The term "palmitoylethanolamide (or PEA) in a micronized form" means PEA having a particle size distribution, defined as a percentage by volume and measured with the laser light scattering method, represented by a distribution curve having the mode between 6 microns and 10 microns.

The term "palmitoylethanolamide (or PEA) in an ultra-micronized form" means PEA having a particle size distribution, defined as a percentage by volume and measured with the laser light scattering method, represented by a distribution curve having the mode below 6 microns and above 0.5 microns.

Preferably, the PEA is in an ultra-micronized form.

In an embodiment, the PEA in an ultra-micronized form has a particle size distribution as defined above, measured with a Malvern Mastersizer 3000 instrument with Fraunhofer calculation algorithm, where at least 95% by volume, more preferably at least 99% by volume, of particles has a particle size of less than 6 microns.

In a particularly preferred embodiment, the PEA in an ultra-micronized form has a particle size distribution as defined above, measured with a Malvern Mastersizer 3000 instrument with Fraunhofer calculation algorithm, having a mode between 2 and 4 microns and having 100% by volume of particles smaller than 10 microns and at least 60% by volume of particles smaller than 3 microns.

The micronization can be carried out in a fluid jet system (for example, Jetmill^{®} model system) which operates with spiral technology with a compressed air or nitrogen jet capable of exploiting kinetic energy - instead of mechanical energy - to crush the particles. Such apparatuses are conventional and will therefore not be further described, except in relation to the following features:
- Internal diameter of the micronization chamber about 300 mm;
- Fluid jet pressure 10-12 bar;
- Product supply 9-12 kg/h.

Docosahexaenoic acid (DHA) belongs to the so-called PUFAs or long-chain polyunsaturated fatty acids and has the following structural formula:

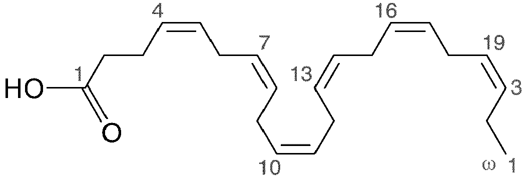

Docosahexaenoic acid (DHA), also called cervonic acid, is an omega-3 or PUFA n-3 fatty acid. Oceanic cold-water fish are rich in DHA. Most of the DHA present in fish and complex organisms, which live in cold ocean waters, comes from photosynthetic algae. DHA is also commercially produced by microalgae, *Crypthecodinium cohnii* which is a microorganism of the genus *Schizochytrium.* DHA produced using microalgae is of plant origin.

The present invention further relates to a composition comprising palmitoylethanolamide and docosahexaenoic acid. Preferably, the composition of the invention consists of a mixture of palmitoylethanolamide and docosahexaenoic acid and pharmaceutically acceptable excipients. More preferably, palmitoylethanolamide is in an ultra-micronized form (PEA-um).

Whether administered separately or combined in a single formulation, PEA and DHA are administered in a weight ratio between 1:7 and 7:1.

More in particular, when PEA is in an ultra-micronized form, the PEA/DHA weight ratio will preferably be between 1:7 and 1:1, more preferably between 1:5 and 1:2.

When the PEA is in a micronized or non-micronized form, the PEA/DHA weight ratio will preferably be between 1:1 and 7:1, more preferably between 2:1 and 5:1.

For the purposes of the invention, PEA alone, DHA alone or the composition containing PEA and DHA can be included in pharmaceutical or veterinary formulations and can be formulated in dosage forms for oral, buccal, parenteral, rectal or transdermal administration.

For oral administration, the compounds of the invention can be found, for example, in the form of tablets or capsules, hard or soft, prepared in the conventional fashion with pharmaceutically acceptable excipients such as binders (e.g., pregelatinized cornstarch, polyvinylpyrrolidone or hydroxypropyl methylcellulose); fillers(e.g., lactose, microcrystalline cellulose or calcium hydrogen phosphate); lubricants (e.g., magnesium stearate, talc or silica); disintegrants (e.g., potato starch or sodium starch glycolate); or inhibiting agents (e.g., sodium lauryl sulfate). The tablets can be coated by methods well known in the art. The liquid preparations for oral administration can be, for example, in the form of solutions, syrups or suspensions or they can be freeze-dried or granulated products to be reconstituted, before use, with water or other suitable vehicles. Such liquid preparations can be prepared through conventional methods with pharmaceutically acceptable additives such as suspending agents (e.g., sorbitol syrup, cellulose derivatives, or edible hydrogenated fats); emulsifiers (e.g., lecithin or acacia); non-aqueous vehicles (e.g., almond oil, oily esters, ethyl alcohol or fractionated vegetable oils); and preservatives (e.g., methyl- or propyl-p-hydroxybenzoates or sorbic acid). The preparation can also conveniently contain flavorings, dyes, and sweeteners.

The preparations for oral administration can be appropriately formulated to allow the controlled release of the active constituent.

For buccal administration, the compounds of the invention can be in the form of tablets or pills formulated in the conventional manner, which are suitable for an absorption at the level of the buccal mucosa. Typical buccal formulations are tablets for sublingual administration.

The compounds of the invention can be formulated for parenteral administration by injection. The injection formulations can be presented as a single dose, for example in vials, with an added preservative. The compositions can appear in such a form as suspensions, solutions, or emulsions in oily or aqueous vehicles and can contain agents of the formulation such as suspension, stabilizers and/or dispersants. Alternatively, the active ingredient or the mixture of active ingredients can be found in the form of a powder to be reconstituted, before use, with a suitable vehicle, for example with sterile water.

The compounds of the invention can also be formulated according to rectal formulations such as suppositories or retention enemas, for example containing the basic components of the common suppositories such as cocoa butter or other glycerides.

In addition to the formulations described above, the compounds of the invention can also be formulated as a deposit preparation. Such long-acting formulations can be administered by implantation (e.g., subcutaneously, transcutaneously or intramuscularly) or intramuscular injection. Therefore, for example, the composition can be formulated with appropriate polymer or hydrophobic materials (for example in the form of an emulsion in a suitable oil) or ion exchange resins or as minimally soluble derivatives.

According to the present invention the daily dose of PEA proposed for administration to a man (with body weight of about 70 kg) ranges from 10 mg to 1500 mg or, if PEA is used in an ultra-micronized form, from 10 mg to 500 mg of PEA. Such a daily dose can be divided into dose units for an administration, for example, from 1 to 4 times a day. The dose will depend on the form in which the PEA is administered, i.e., whether non-micronized PEA, micronized PEA or ultra-micronized PEA is administered. The dose will also depend on the route chosen for administration. It should be considered that it may be necessary to continuously vary the dosage depending on the age and weight of the patient and also on the severity of the clinical condition to be treated. The exact dose and route of administration will ultimately be at the discretion of the treating physician or veterinarian.

The invention further relates to dietary compositions, food supplements and foods for special medical purposes (FSMPs) comprising PEA, preferably ultra-micronized PEA, and DHA for use in the treatment of ASD.

The term "food for special medical purposes" means products authorized according to regulation (EU) 2016/128. Such a term refers to a product to be administered under medical supervision, thus assimilating such an FSMP to a drug.

The formulations according to the invention can be prepared according to conventional methods, such as those described in Remington's Pharmaceutical Sciences Handbook, Mack Pub. Co., N.Y., USA, 17th edition, 1985 or in Remington, The Science and Practice of Pharmacy, Edited by Allen, Loyd V., Jr, 22nd edition, 2012.

### EXPERIMENTAL SECTION

### Micronization procedure

The PEA was micronized as previously described.

The ultra-micronization was carried out in a fluid jet system (in particular, the Jetmill^{®} model system) which operates with compressed air jet "spiral technology".

Optimal micronization conditions:
- internal diameter of the micronization chamber 300 mm;
- fluid jet pressure 8 bar;
- product supply 9-12 kg/h.

### Determination of the particle size distribution

The determination of the particle size distribution was carried out on a wet sample, after 1-minute sonication.

A Malvern Mastersizer 3000 instrument operating with the LALLS (Low Angle Laser Light Scattering) technique and a Fraunhofer calculation algorithm was used.

The particle size distribution graph is shown in figure 1.

### Biological experimentation

Healthy male C57BL/6J (C57) mice and 90-day BTBR T+tf/J (BTBR) mice (The Jackson Laboratory, Bar Harbor, ME, USA) fed *ad libitum* and housed in controlled sleep/wake cycle cages were used in the *in vivo* trial. Before the start of the experimentation, the animals were subjected to an acclimatization period of 1 week considering all the experimental procedures and protocols, compliant with the principles of care and welfare of laboratory animals approved by the Italian Ministry of Health (Italian Legislative Decree 2014/26) and European directives (EU Directive 2010/63).

BTBR mice have a simil-autistic phenotype capable of reproducing the main symptoms of ASD with the appearance of behavioral deficits in a period comparable to early childhood. By virtue of the numerous polymorphisms due to the mutations of individual nucleotides involved in the development of the nervous system and synapses, BTBR mice are completely free of the corpus callosum and subject to severely reduced hippocampal commissure (Wahlsten D. et al., Survey of 21 inbred mouse strains in two laboratories reveals that BTBR T/+ tf/tf has severely reduced hippocampal commissure and absent corpus callosum, Brain Res. 2003, 971: 47-54). This strain has several symptoms of autism including reduced social interactions, altered expressions of play, reduced exploratory behavior, unusual vocalizations, and anxiety (McFarlane HG et al., Autism-like behavioral phenotypes in BTBR T+tf/J mice, Genes Brain Behav. 2008, 7: 152-63; Scattoni ML et al., Unusual repertoire of vocalizations in the BTBR T+tf/J mouse model of autism, PLoS One 2008, 3: e3067); further has particularly reduced ALLO levels (Ebihara K. et al., Decrease in endogenous brain allopregnanolone induces autism spectrum disorder (ASD)-like behavior in mice: A novel animal model of ASD, Behav Brain Res. 2017, 334: 6-15; Chew L. et al., Association of serum allopregnanolone with restricted and repetitive behaviors in adult males with autism Psychoneuroendocrinology, 2021, 123: 105039).

### Experimental methods and results

Healthy C57 animals and BTBR animals were randomized into 8 groups of 8 mice each and treated *per os,* starting from the fourth month of life, with 1.5% carboxymethyl cellulose (CMC) (vehicle used to suspend molecules), with 1 mg/kg ultra-micronized PEA (PEA-um) alone, with 5 mg/kg DHA alone (30 mg/kg DHA 17% titer) and with 1 mg/kg PEA-um associated with 5 mg/kg DHA (30 mg/kg DHA 17% titer), daily for 10 days:
- Group 1: C57 mice treated with 1.5% CMC as control (CTR) ;
- Group 2: C57 mice treated with 1 mg/kg PEA-um suspended in 1.5% CMC (PEA);
- Group 3: C57 mice treated with 5 mg/kg DHA (30 mg/kg DHA 17% titer) suspended in 1.5% CMC (DHA);
- Group 4: C57 mice treated with 1 mg/kg PEA-um and 5 mg/kg DHA (30 mg/kg DHA 17% titer) suspended in 1.5% CMC (PEA+DHA composition);
- Group 5: BTBR mice treated with 1.5% CMC (CTR);
- Group 6: BTBR mice treated with 1 mg/kg PEA-um suspended in 1.5% CMC (PEA);
- Group 7: BTBR mice treated with 5 mg/kg DHA (30 mg/kg DHA 17% titer) suspended in 1.5% CMC (DHA);
- Group 8: BTBR mice treated with 1 mg/kg PEA-um and 5 mg/kg DHA (30 mg/kg DHA 17% titer) suspended in 1.5% CMC (PEA+DHA composition).

The animals were euthanized 10 days after starting the administration of the treatments. Plasma was collected for dosing with the HLPC (Agilent) method of the ALLO neurosteroid. Before being sacrificed, the animals were subjected to behavioral tests to study the repetitive/obsessive phenotype (Marble Burying test and Self Grooming test) and sociability.

All the behavioral tests were conducted by the same mice with a sufficient time interval between one test and the other and starting from the least stressful one (Paylor R. et al., The use of behavioral test batteries, II: effect of test interval, Physiol. Behav. 2006, 87: 95-102) .

### Statistical analysis

All the values reported in the results are expressed as mean ± standard error of the mean (SEM) of N observations (N = number of animals). The statistical differences in the ALLO dosage and behavioral score were analyzed with one-way ANOVA, followed by Sidak's multiple comparisons. P-value <0.05 is considered significant.

### Detection of obsessive and repetitive behavior

In the Marble Burying test, 20 marbles were placed in a grid inside a plexiglass cage filled with 5 cm of clean litter. Each mouse was placed in the cage and, after 15 session minutes, gently removed to count the number of buried marbles. The BTBR mice treated with only vehicle (CTR), only PEA-um 1 mg/kg (PEA) and only DHA 30 mg/kg (DHA), obsessively bury the marbles. Conversely, the treatment with PEA-um 1 mg/kg administered in combination with DHA 30 mg/kg significantly reduces the obsessive attitude of mice in hiding the marbles. As proof of the above, all the C57 animals (healthy animals) subjected to the treatments did not show significant changes in the number of buried marbles (Fig. 2A).

In the Self Grooming test, the mice were placed in an empty plexiglass cage (30x40 cm) and allowed to freely explore the arena. After 10 minutes of acclimatization, self grooming activity was monitored for 20 minutes. The repetitive attitude in washing the head, body, genital area and tail and in licking the arms and legs were taken into account.

Only treatment with PEA+DHA administered in combination is capable of reducing the seconds spent self-grooming by BTBR mice. The animals with ASD of the CTR, PEA and DHA groups did not demonstrate any reduction in the time (s) spent self-grooming (Fig. 2B). Again, in the C57 mice (healthy animals), no differences are observed between controls and treated animals.

### Sociability of the animals

Social interaction was examined using a three-chamber instrument. This test consists of 3 phases: in the first phase the animal is acclimatized for 5 minutes in the empty arena (center). In the next 10-minute session, the animal is exposed to an empty chamber on the left side of the instrument or to an unknown mouse in the right chamber.

In the last 10-minute phase, the preference of the mouse in staying in the empty chamber or in the presence of the mouse was evaluated (Crawley JN, Designing mouse behavioral tasks relevant to autistic-like behaviors, Ment. Retard Dev. Disabil. Res. Rev. 2004, 10: 248-258). The time spent in each chamber was detected by a camera coupled to video tracking software.

The healthy C57 mice exhibit enhanced sociability to another mouse also following treatments with either only PEA-um or only DHA at the inactive concentrations of 1 mg/kg and 30 mg/kg (Fig. 3A). Conversely, in the BTBR mice, the sociability of which is reduced, only the synergy between PEA-um associated with DHA improves the sociability of autistic animals, leading the mice to spend more time in the company of a companion. The BTBR animals treated with vehicle, only PEA-um 1 mg/kg and only DHA 30 mg/kg did not demonstrate social improvements, continuing to spend their time in the empty side of the instrument (Fig. 3B).

### Synergy between PEA-um associated with DHA increases endogenous ALLO neurosteroid levels in ASD mice

BTBR mice have a significant reduction in plasma ALLO levels; this trend is also found in BTBR animals treated with PEA-um 1 mg/kg and DHA 30 mg/kg. Only the group of BTBR animals treated with the PEA-um 1 mg/kg + DHA 30 mg/kg combination has a significant increase in plasma levels of the neurosteroid ALLO. All the treatments performed in the healthy C57 mice did not induce any plasma ALLO increase (Fig. 4).

From the above demonstration, the synergistic effect resulting from the association between PEA and DHA allows to use lower dosages of both active substances than those normally used when such active ingredients are administered alone or, in the case of DHA, with other PUFAs, such as EPA.

The present invention thus provides an agent for use in a method for treating autism spectrum disorders (ASD) comprising or consisting in administering, to a subject suffering from ASD, said agent comprising

PEA, preferably PEA in an ultra-micronized form, and DHA, where said administration is separate, combined (i.e., in a single dosage form) or simultaneous and where PEA and DHA are administered at a dosage at which, when administered alone, PEA and DHA are inactive.

Preferably, the doses of PEA-um and DHA administered to a child or adolescent patient are 500 mg/day or less and 700 mg/day, or more preferably 300 mg/day or less and 500 mg/day or less, respectively.

Furthermore, the present invention provides a method for increasing endogenous allopregnanolone levels in a subject in which said endogenous levels are below normal levels (i.e., preferably below 0.7 nmol/L), comprising or consisting in administering to said subject PEA, preferably in an ultra-micronized form, and DHA, where said administration is separate, combined (i.e., in a single dosage form) or simultaneous and where PEA and DHA are administered at a dosage at which, when administered alone, PEA and DHA are inactive.

Therefore, such a method allows the treatment not only of subjects with ASD, but also of subjects with depressive syndromes, in particular postpartum depression.

The invention will now be further described by means of the following formulation examples.

### Formulation examples

### PEA-um = Ultra-micronized palmitoylethanolamide

### Example 1 - Soft gelatin capsules

### 12-Twist-off capsule content:

| | |
|---|---|
| PEA-um | 150.00 mg |
| DHA (titer 55%) | 435.00 mg |
| Peanut oil | 40.00 mg |
| Soy lecithin | 20.00 mg |
| Alpha-tocopherol | 10.00 mg |
| Glyceryl monostearate | 10.00 mg |

| Composition of the capsule: | |
|---|---|
| Bovine gelatin | 237.00 mg |
| glycerol | 130.00 mg |
| Water | 19.00 mg |
| Pigments | 0.07 mg |

### Example 2 - Syrup

### Composition per 100 ml:

| | |
|---|---|
| Sucrose | 25.0 g |
| Palmitoylethanolamide-m | g 12.0 |
| DHA with 55% titer | 5.0 g |
| Microcrystalline cellulose | 1.35 g |
| Natural tocopherol (1000IU/g) | 1.0 g |
| Sodium Carboxymethyl cellulose | 0.65 g |
| Sorbitan monooleate | 0.40 g |
| Polysorbate 80 | 0.10 g |
| Natural flavoring | 0.10 g |
| Potassium sorbate | 0.09 g |
| Benzoic acid | 0.07 g |
| Citric acid | 0.05 g |
| Water | q.s. to 100ml |

### Example 3 - Dispersible granules

### Contents of the single-dose sachet:

| | |
|---|---|
| Palmitoylethanolamide | 250 mg |
| DHA powder with 17% titer | 1400 mg |
| Maltodextrin | 500 mg |
| Fructose | 300 mg |
| Dextrose | 200 mg |
| Tocopherol acetate 50% (powder on silica) | 200 mg |
| Citric acid | 50 mg |
| Pluronic F-68 | 50 mg |
| Natural flavoring | 50 mg |
| Magnesium Stearate | 10 mg |
| Polysorbate 80 | 10 mg |

## Claims

1. Palmitoylethanolamide for use in the treatment of autism spectrum disorders (ASDs), wherein palmitoylethanolamide is administered in association with docosahexaenoic acid (DHA), wherein said administration is separate, combined, or simultaneous.

2. Palmitoylethanolamide for use according to claim 1, wherein palmitoylethanolamide is in a non-micronized form, having a particle size distribution, defined as percentage by volume and measured by the laser light scattering method, represented by a distribution curve having the mode above 10 microns, preferably above 20 microns.

3. Palmitoylethanolamide for use according to claim 1, wherein palmitoylethanolamide is in a micronized form, having a particle size distribution, defined as percentage by volume and measured by the laser light scattering method, represented by a distribution curve having the mode between 6 microns and 10 microns.

4. Palmitoylethanolamide for use according to claim 1, wherein palmitoylethanolamide is in an ultra-micronized form having a particle size distribution, defined as percentage by volume and measured by the laser light scattering method, represented by a distribution curve having the mode below 6 microns and above 0.5 microns.

5. Palmitoylethanolamide for use according to claim 4, having a particle size distribution, defined as percentage by volume and measured by the laser light scattering method, measured with a Malvern Mastersizer 3000 instrument with Fraunhofer calculation algorithm, wherein at least 95% by volume, preferably at least 99% by volume, of particles has a particle size less than 6 microns.

6. Palmitoylethanolamide for use according to claim 4, wherein palmitoylethanolamide has a particle size distribution, defined as a percentage by volume and measured by the laser light scattering method, measured with a Malvern Mastersizer 3000 instrument with Fraunhofer calculation algorithm, having a mode between 2 and 4 microns and having 100% by volume of particles less than 10 microns and at least 60% by volume of particles less than 3 microns.

7. Palmitoylethanolamide for use according to any one of claims 1 to 6, wherein PEA and DHA are administered in a weight ratio between 1:7 and 7:1.

8. Palmitoylethanolamide for use according to any one of claims 4 to 6, wherein the PEA/DHA weight ratio is between 1:7 and 1:1, preferably between 1:5 and 1:2.

9. Palmitoylethanolamide for use according to any one of claims 1 to 8, wherein the daily dose of PEA for administration to a subject ranges from 10 mg to 1500 mg or, if PEA in an ultra-micronized form is used, from 10 mg to 500 mg of PEA, or the dosages of PEA and DHA for a child or adolescent subject are equal to or less than 500 mg/day and 700 mg/day or preferably equal to or less than 300 mg/day and 500 mg/day, respectively.

10. Palmitoylethanolamide for use according to any one of claims 1 to 9, wherein palmitoylethanolamide and DHA are contained in pharmaceutical or veterinary formulations and are formulated in dosage forms for oral, buccal, parenteral, rectal, or transdermal administration.

11. Palmitoylethanolamide for use according to any one of claims 1 to 9, wherein palmitoylethanolamide and DHA are contained in dietary compositions, food supplements, or foods for special medical purposes (FSMPs).

12. Palmitoylethanolamide for use in the treatment of diseases **characterized by** endogenous plasma levels of allopregnanolone which are lower than 0.7 nmol/L, which comprises or consists in administering PEA, preferably in an ultra-micronized form, and DHA to said subjects, wherein said administration is separate, combined, or simultaneous.

13. Palmitoylethanolamide according to claim 12, for use in the treatment of depressive syndromes, preferably of postpartum depression.

14. A composition comprising or consisting of a mixture of palmitoylethanolamide, preferably ultra-micronized palmitoylethanolamide, and docosahexaenoic acid and pharmaceutically acceptable excipients, for use in the treatment of autism spectrum disorders (ASDs) or of depressive syndromes, preferably of postpartum depression, wherein palmitoylethanolamide and docosahexaenoic acid are contained in a weight ratio between 1:7 and 7:1 or, when palmitoylethanolamide is in an ultra-micronized form, in a weight ratio between 1:7 and 1:1, preferably between 1:5 and 1:2.

15. A pharmaceutical or veterinary formulation, dietary compositions, food supplements, or foods for special medical purposes for use in the treatment of autism spectrum disorders (ASDs) or of depressive syndromes, preferably of postpartum depression, comprising the composition as defined in claim 14.

## Patentansprüche

1. Palmitoylethanolamid zur Verwendung bei der Behandlung von Autismus-Spektrum-Störungen (ASDs), wobei Palmitoylethanolamid in Verbindung mit Docosahexaensäure (DHA) verabreicht wird, wobei die Verabreichung getrennt, kombiniert oder gleichzeitig erfolgt.

2. Palmitoylethanolamid zur Verwendung nach Anspruch 1, wobei Palmitoylethanolamid in einer nicht-mikronisierten Form vorliegt, mit einer Partikelgrößenverteilung, definiert als Volumenprozent und gemessen durch das Laserlichtstreuungsverfahren, dargestellt durch eine Verteilungskurve mit dem Modus über 10 Mikron, vorzugsweise über 20 Mikron.

3. Palmitoylethanolamid zur Verwendung nach Anspruch 1, wobei Palmitoylethanolamid in einer mikronisierten Form vorliegt, mit einer Partikelgrößenverteilung, definiert als Volumenprozentsatz und gemessen durch das Laserlichtstreuungsverfahren, dargestellt durch eine Verteilungskurve mit dem Modus zwischen 6 Mikron und 10 Mikron.

4. Palmitoylethanolamid zur Verwendung nach Anspruch 1, wobei Palmitoylethanolamid in einer ultra-mikronisierten Form vorliegt, mit einer Partikelgrößenverteilung, definiert als Volumenprozent und gemessen durch das Laserlichtstreuungsverfahren, dargestellt durch eine Verteilungskurve mit dem Modus unter 6 Mikron und über 0,5 Mikron.

5. Palmitoylethanolamid zur Verwendung nach Anspruch 4, mit einer Partikelgrößenverteilung, definiert als Volumenprozent und gemessen durch das Laserlichtstreuungsverfahren, gemessen mit einem Malvern Mastersizer 3000 Instrument mit Fraunhofer-Kalkulationsalgorithmus, wobei mindestens 95 Volumenprozent, vorzugsweise mindestens 99 Volumenprozent, der Partikel eine Partikelgröße von weniger als 6 Mikron aufweisen.

6. Palmitoylethanolamid zur Verwendung nach Anspruch 4, wobei Palmitoylethanolamid eine Partikelgrößenverteilung aufweist, definiert als Volumenprozentsatz und gemessen durch das Laserlichtstreuungsverfahren, gemessen mit einem Malvern Mastersizer 3000 Instrument mit Fraunhofer-Kalkulationsalgorithmus, mit einem Modus zwischen 2 und 4 Mikron und mit 100 Volumenprozent der Partikel weniger als 10 Mikron und mindestens 60 Volumenprozent der Partikel weniger als 3 Mikron.

7. Palmitoylethanolamid zur Verwendung nach einem der Ansprüche 1 bis 6, wobei PEA und DHA in einem Gewichtsverhältnis zwischen 1:7 und 7:1 verabreicht werden.

8. Palmitoylethanolamid zur Verwendung nach einem der Ansprüche 4 bis 6, wobei das PEA/DHA-Gewichtsverhältnis zwischen 1:7 und 1:1, vorzugsweise zwischen 1:5 und 1:2, liegt.

9. Palmitoylethanolamid zur Verwendung nach einem der Ansprüche 1 bis 8, wobei die Tagesdosis von PEA zur Verabreichung an ein Individuum im Bereich von 10 mg bis 1500 mg liegt oder, wenn PEA in einer ultra-mikronisierten Form verwendet wird, im Bereich von 10 mg bis 500 mg PEA liegt, oder die Dosierungen von PEA und DHA für ein Kind oder jugendliches Individuum jeweils gleich oder weniger als 500 mg/Tag und 700 mg/Tag oder vorzugsweise gleich oder weniger als 300 mg/Tag und 500 mg/Tag betragen.

10. Palmitoylethanolamid zur Verwendung nach einem der Ansprüche 1 bis 9, wobei Palmitoylethanolamid und DHA in pharmazeutischen oder veterinärmedizinischen Formulierungen enthalten sind und in Darreichungsformen zur oralen, bukkalen, parenteralen, rektalen oder transdermalen Verabreichung formuliert sind.

11. Palmitoylethanolamid zur Verwendung nach einem der Ansprüche 1 bis 9, wobei Palmitoylethanolamid und DHA in diätetischen Zusammensetzungen, Nahrungsergänzungsmitteln oder Lebensmitteln für besondere medizinische Zwecke (FSMPs) enthalten sind.

12. Palmitoylethanolamid zur Verwendung bei der Behandlung von Krankheiten, welche durch endogene Plasmaspiegel von Allopregnanolon gekennzeichnet sind, welche geringer als 0,7 nmol/L sind, welche Verabreichung von PEA, vorzugsweise in einer ultra-mikronisierten Form, und DHA an die Individuen umfasst oder darin besteht, wobei die Verabreichung getrennt, kombiniert oder gleichzeitig erfolgt.

13. Palmitoylethanolamid nach Anspruch 12, zur Verwendung bei der Behandlung von depressiven Syndromen, vorzugsweise von postpartaler Depression.

14. Zusammensetzung, umfassend oder bestehend aus einem Gemisch von Palmitoylethanolamid, vorzugsweise ultra-mikronisiertem Palmitoylethanolamid, und Docosahexaensäure und pharmazeutisch akzeptablen Hilfsstoffen, zur Verwendung bei der Behandlung von Autismus-Spektrum-Störungen (ASDs) oder von depressiven Syndromen, vorzugsweise von postpartaler Depression, wobei Palmitoylethanolamid und Docosahexaensäure in einem Gewichtsverhältnis zwischen 1:7 und 7:1 oder, wenn Palmitoylethanolamid in ultra-mikronisierter Form vorliegt, in einem Gewichtsverhältnis zwischen 1:7 und 1:1, vorzugsweise zwischen 1:5 und 1:2 enthalten sind.

15. Pharmazeutische oder veterinärmedizinische Formulierung, diätetische Zusammensetzungen, Nahrungsergänzungsmittel oder Lebensmittel für besondere medizinische Zwecke zur Verwendung bei der Behandlung von Autismus-Spektrum-Störungen (ASD) oder von depressiven Syndromen, vorzugsweise von postpartaler Depression, umfassend die in Anspruch 14 definierte Zusammensetzung.

## Revendications

1. Palmitoyléthanolamide à utiliser dans le traitement de troubles du spectre autistique (TSA), dans lequel le palmitoyléthanolamide est administré en association avec l'acide docosahexaénoïque (DHA), ladite administration étant séparée, combinée ou simultanée.

2. Palmitoyléthanolamide à utiliser selon la revendication 1, dans lequel le palmitoyléthanolamide se présente sous une forme non micronisée, présentant une répartition de tailles de particules, définie en pourcentage en volume et mesurée selon le procédé de diffraction laser, représentée par une courbe de répartition ayant le mode supérieur à 10 microns, de préférence supérieur à 20 microns.

3. Palmitoyléthanolamide à utiliser selon la revendication 1, dans lequel le palmitoyléthanolamide se présente sous une forme micronisée, présentant une répartition de tailles de particules, définie en pourcentage en volume et mesurée selon le procédé de diffraction laser, représentée par une courbe de répartition ayant le mode entre 6 microns et 10 microns.

4. Palmitoyléthanolamide à utiliser selon la revendication 1, dans lequel le palmitoyléthanolamide se présente sous une forme ultra-micronisée présentant une répartition de tailles de particules, définie en pourcentage en volume et mesurée selon le procédé de diffraction laser, représentée par une courbe de répartition ayant le mode inférieur à 6 microns et supérieur à 0,5 micron.

5. Palmitoyléthanolamide à utiliser selon la revendication 4, présentant une répartition de tailles de particules, définie en pourcentage en volume et mesurée selon le procédé de diffraction laser, mesurée avec un instrument Malvern Mastersizer 3000 avec l'algorithme de calcul Fraunhofer, dans lequel au moins 95 % en volume, de préférence au moins 99 % en volume, des particules ont une taille de particules inférieure à 6 microns.

6. Palmitoyléthanolamide à utiliser selon la revendication 4, dans lequel le palmitoyléthanolamide présente une répartition de tailles de particules, définie en pourcentage en volume et mesurée selon le procédé de diffraction laser, mesurée avec un instrument Malvern Mastersizer 3000 avec l'algorithme de calcul Fraunhofer, présentant un mode entre 2 et 4 microns et présentant 100 % en volume de particules inférieures à 10 microns et au moins 60 % en volume de particules inférieures à 3 microns.

7. Palmitoyléthanolamide à utiliser selon l'une quelconque des revendications 1 à 6, dans lequel le PEA et le DHA sont administrés selon un rapport pondéral entre 1:7 et 7:1.

8. Palmitoyléthanolamide à utiliser selon l'une quelconque des revendications 4 à 6, dans lequel le rapport pondéral PEA/DHA est entre 1:7 et 1:1, de préférence entre 1:5 et 1:2.

9. Palmitoyléthanolamide à utiliser selon l'une quelconque des revendications 1 à 8, dans lequel la dose quotidienne de PEA à administrer à un sujet va de 10 mg à 1500 mg ou, si du PEA sous forme ultra-micronisée est utilisé, de 10 mg à 500 mg de PEA, ou les dosages de PEA et de DHA pour un sujet enfant ou adolescent sont respectivement inférieurs ou égaux à 500 mg/jour et 700 mg/jour ou de préférence inférieurs ou égaux à 300 mg/jour et 500 mg/jour.

10. Palmitoyléthanolamide à utiliser selon l'une quelconque des revendications 1 à 9, dans lequel le palmitoyléthanolamide et le DHA sont contenus dans des formulations pharmaceutiques ou vétérinaires et sont formulés sous des formes galéniques pour une administration orale, buccale, parentérale, rectale ou transdermique.

11. Palmitoyléthanolamide à utiliser selon l'une quelconque des revendications 1 à 9, dans lequel le palmitoyléthanolamide et le DHA sont contenus dans des compositions diététiques, des compléments alimentaires, ou des aliments destinés à une alimentation particulière à des fins médicales spéciales (ADFMS).

12. Palmitoyléthanolamide à utiliser dans le traitement de maladies **caractérisées par** des concentrations plasmatiques endogènes d'alloprégnanolone qui sont inférieures à 0,7 nmol/l, qui comprend ou consiste en l'administration de PEA, de préférence sous une forme ultra-micronisée, et de DHA auxdits sujets, ladite administration étant séparée, combinée ou simultanée.

13. Palmitoyléthanolamide selon la revendication 12, à utiliser dans le traitement de syndromes dépressifs, de préférence de la dépression post-partum.

14. Composition comprenant ou consistant en un mélange de palmitoyléthanolamide, de préférence de palmitoyléthanolamide ultramicronisé, et d'acide docosahexaénoïque et d'excipients pharmaceutiquement acceptables, à utiliser dans le traitement de troubles du spectre autistique (TSA) ou de syndromes dépressifs, de préférence la dépression post-partum, dans laquelle le palmitoyléthanolamide et l'acide docosahexaénoïque sont contenus selon un rapport pondéral entre 1:7 et 7:1 ou, lorsque le palmitoyléthanolamide se présente sous une forme ultramicronisée, selon un rapport pondéral entre 1:7 et 1:1, de préférence entre 1:5 et 1:2.

15. Formulation pharmaceutique ou vétérinaire, compositions diététiques, compléments alimentaires ou aliments destinés à une alimentation particulière à des fins médicales spéciales, à utiliser dans le traitement de troubles du spectre autistique (TSA) ou de syndromes dépressifs, de préférence la dépression post-partum, et comprenant la composition telle que définie dans la revendication 14.
